# EUROPÄISCHE PATENTSCHRIFT

(11) **EP 1 467 782 B1**
(45) Veröffentlichungstag und Bekanntmachung des Hinweises auf die Patenterteilung: **14.04.2010**
(21) Anmeldenummer: 02769837.2
(22) Anmeldetag: 06.11.2002
(51) Int. Cl.: A61M 5/142, F04B 43/09

(54) **IMPLANTIERBARE INFUSIONSPUMPE**
IMPLANTABLE INFUSION PUMP
POMPE A PERFUSION IMPLANTABLE

(30) Priorität: 06.11.2001 CH 203401
(43) Veröffentlichungstag der Anmeldung: 20.10.2004
(73) Patentinhaber: Keller, Hermann L., 4052 Basel (CH)
(72) Erfinder: Keller, Hermann L., 4052 Basel (CH)
(74) Vertreter: Wagner, Kilian
(86) Internationale Anmeldenummer: PCT/CH2002/000596
(87) Internationale Veröffentlichungsnummer: WO 2003/039631

(56) Entgegenhaltungen:
- WO-A-00/66202
- DE-A- 2 421 433
- US-A- 4 519 751
- US-A- 4 596 575
- US-A- 4 822 250
- US-A- 4 944 659
- US-A- 5 113 859
- US-A- 5 569 187

## Beschreibung

Die Erfindung betrifft eine Infusionspumpe nach dem Oberbegriff des Patentanspruches 1.

Zur Infusion - unter Infusion wird die Einführung von Flüssigkeiten beispielsweise in einen menschlichen oder tierischen Körper verstanden - sind verschiedene Verfahren und Vorrichtungen bekannt. Ein bekanntes Verfahren ist die unter Ausnutzung der Schwerkraft erfolgende intravenöse Einleitung einer Flüssigkeit ausgehend von einem Behälter, der bestimmte Flüssigkeitsmengen pro Zeiteinheit freisetzt, die über eine Förderleitung, gewöhnlich einem Schlauch, einer Vene zugeführt werden. Dieses Verfahren und seine zur Durchführung bestimmte Vorrichtung sind unter der Kurzbezeichnung "Tropf" bekannt, er ist technisch gesehen anspruchslos, funktionssicher, eignet sich aber nur zur Anwendung bei Patienten, deren Bewegungsfreiheit Einschränkungen unterliegt.

Um diese Einschränkungen zu beseitigen, sind sogenannte Dosierpumpen (folgend kurz Pumpen genannt) entwickelt und zur Anwendung gebracht worden, die der Rezipient des Fördermediums zum Erhalt seiner Bewegungsfreiheit ständig auf sich trägt. Pumpen umfassen im wesentlichen ein Gehäuse, in dem ein nachfüllbarer Medien- d.h. Flüssigkeits- oder Medikamentenbehälter und eine Einrichtung zur Förderung des Mediums zum Anschluss einer Förderleitung abgehend von besagter Einrichtung aufgenommen sind. Die Förderleitung - auch Katheter genannt - kann beispielsweise im Spinalraum oder in der Blutbahn je nach medizinischer Indikation des Rezipienten ausmünden.

Gemäss ihrer Ausbildung unterscheidet man bei Pumpen zwischen "variable Flow Pumps" (kurz VF Pumpen) und "constant Flow Pumps" (kurz CF Pumpen). VF Pumps sind Pumpen, deren Fördermenge pro Zeiteinheit einstellbar ist, während die Fördermenge pro Zeiteinheit bei CF Pumpen unveränderbar, d.h. konstant, ist. Die VF Pumpe weist zur Förderung des Mediums vom Vorratsbehälter zum Anschluss eines Katheters als mechanische Einrichtung eine batteriegetriebene Rollenpumpe auf, deren Betriebsparameter wie Drehzahl, Fördermenge etc. elektronisch über einen Chip veränderbar einstellbar sind. Dabei erfolgt die Veränderung der Betriebsparameter durch Uebertragung von Daten einer Datenverarbeitungseinrichtung, z.B. einem Computer via Leitung und Lesekopf auf den Chip. Der Füllstand des Vorratsbehälters und damit der Verbrauch über eine gewisse Zeitdauer wird bei VF Pumpen nicht gemessen sondern errechnet. Dies bedeutet, dass nicht der Füllstand eine Nachfüllung anzeigt, sondern nach Ablauf einer vorgegebenen und auf dem Chip eingegebenen Zeitperiode unabhängig des Restbestandes an Medien im Vorratsbehälter nachzufüllen ist. Berücksichtigt man bei VF Pumpen die relativ grossen Gangungenauigkeiten der Rollenpumpen, d.h. Fördermengenabweichungen von einem eingestellten Fördermengenwert von plus/minus 10% bis 15% so wird deutlich, dass relativ grosse Restmengen an Medien, d.h. Medikamenten, vor Auslösen eines Nachfüllsignales vorzusehen sind. Restmengen sind vor Nachfüllung aus dem Vorratsbehälter abzuziehen und können nicht weiter verwendet werden. Bei kostspieligen Medikamenten verteuert dieser Umstand den Betrieb einer Pumpe nachhaltig. Die einstellbare VF Pumpe kann für sich den Vorteil in Anspruch nehmen, jederzeit bedürfnisgerecht programmierbar zu sein, diesem Vorteil stehen die Nachteile gegenüber, dass die bedürfnisgerechte Programmierung nur von besonders geschulten Medizinern vorgenommen werden kann, ein Patient sich aufgrund der Übertragung vermittels einer Leitung zwischen Computer und Lesekopf zur Datenverarbeitungsanlage begeben muss oder umgekehrt und dass relativ grosse Medikamente-Restmengen als Folge von Gangungenauigkeiten hinzunehmen sind.

Eine CF Pumpe ist im Gegensatz zur VF Pumpe einfacher ausgestaltet. Die Förderung des Mediums aus dem Vorratsbehälter zum Katheteranschluss erfolgt vermittels eines Druckgaspolsters, wobei zwischen Vorratsbehälter und Katheteranschluss eine Reduziereinrichtung vorgesehen ist, die eine Durchflussmenge pro Zeiteinheit unvariabel bestimmt. CF Pumpen werden nach Rezept patientenspezifisch gefertigt, variiert werden kann nur die Zusammensetzung des Fördermediums, nicht jedoch wie bei der VF Pumpe Fördermenge und Zusammensetzung. Der Zeitpunkt der Nachfüllung wird anlässlich einer vorgängigen Füllung aufgrund der bekannten Durchflussmengen bestimmt. Die Fördergenauigkeit, d.h. die Genauigkeit einer Durchflussmenge pro Zeiteinheit ist höher als bei VF Pumpen, so dass sich Restmengenbemessungen nicht mit gleicher Problematik wie bei VF Pumpen stellen. Den Vorteilen des einfachen Aufbaues und einer höheren Fördergenauigkeit im Vergleich zu VF Pumpen und die Möglichkeit der Nachfüllung durch nicht spezialisiertes medizinisches Personal steht mithin der Nachteil begrenzter Einstellmöglichkeiten gegenüber.

Mehrheitlich werden Pumpen der beschriebenen Art Trägern, d.h. Patienten, implantiert. Die Implantation erfolgt im Bauchbereich zwischen Muskeln und Haut, d.h. im subkutanen Fettgewebe. Die Gehäuse der Pumpen sind als Dosen ausgebildet, normalerweise haben diese einen Durchmesser von ca. 8 cm und eine Höhe von ca. 2 cm bis 2,5 cm, das Gewicht der Pumpen liegt in der Grössenordnung von ca. 200 gr. Gestaltung und Gewicht der Pumpen führen nach Implantation zu einem kosmetischen Problem für den Patienten, indem als Folge der Dosenform und des Gewichtes der Implantationsbereich eine deutliche Deformation erfährt.

Aus der US-A-4 944 659, der US-A-5 569 187, der US-A-4 569 575, der US-A-4 822 250 sind Infusionspumpen bekannt. Die DE 24 21 433 A zeigt einen Folienbeutel für Medikamentenflüssigkeiten. Keine der bekannten Infusionspumpen ist an den Implantationsort anpassbar.

Ausgehend von diesem Stand der Technik haben sich die Erfinder die Aufgabe gestellt, eine Pumpe zu schaffen, die die Vorteile der bekannten Pumpen, nicht aber deren Nachteile, auf sich vereinigt und diese Aufgabe wird mit einer Pumpe mit den kennzeichnenden Merkmalen des Patentanspruches 1 gelöst.

Vorteilhafte Ausgestaltungen einer Pumpe nach Patentanspruch 1 kennzeichnen die Merkmale der dem Patentanspruch 1 folgenden Patentansprüche.

Weitere Vorteile, Merkmale und Einzelheiten der Erfindung ergeben sich aus der folgenden Beschreibung eines bevorzugten Ausführungsbeispieles und der Zeichnung, es zeigen:
- Fig. 1: eine Draufsicht auf die obere Schale einer Pumpe,
- Fig. 2: eine Seitenansicht der Pumpe gemäss Fig. 1, angepasst an die Form des Implantationsortes des Körpers eines Patienten,

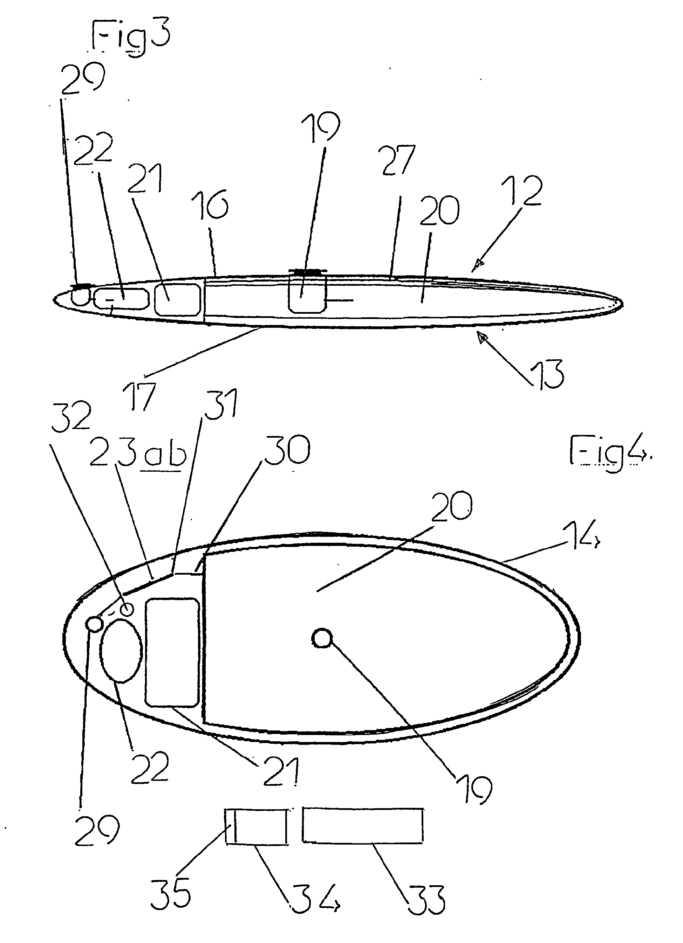

- Fig. 3: einen mittigen Vertikalschnitt durch die Pumpe gemäss Fig. 1 in der Seitenansicht,
- Fig. 4: einen Horizontalschnitt durch die Pumpe ge- mäss Fig. 1 in der Draufsicht, Tank und Tank- belag ergänzt um übrige Bauteile der Pumpe,

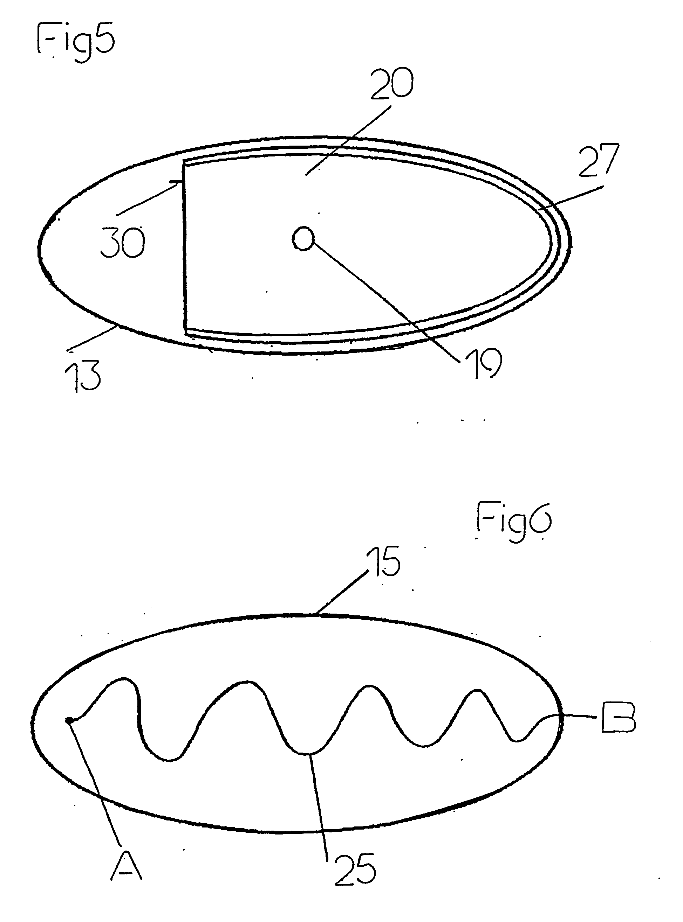

- Fig. 5: einen Horizontalschnitt durch die Pumpe ge- mäss Fig. 1 in der Draufsicht, nur Tank und Tankbelag in der Schnittdarstellung zeigend,
- Fig. 6: eine Draufsicht auf die untere Schale einer Pumpe gemäss Fig. 1.
- Fig. 7: eine schematische Darstellung der Schaltunge 1 bis einer Piezzopumpe

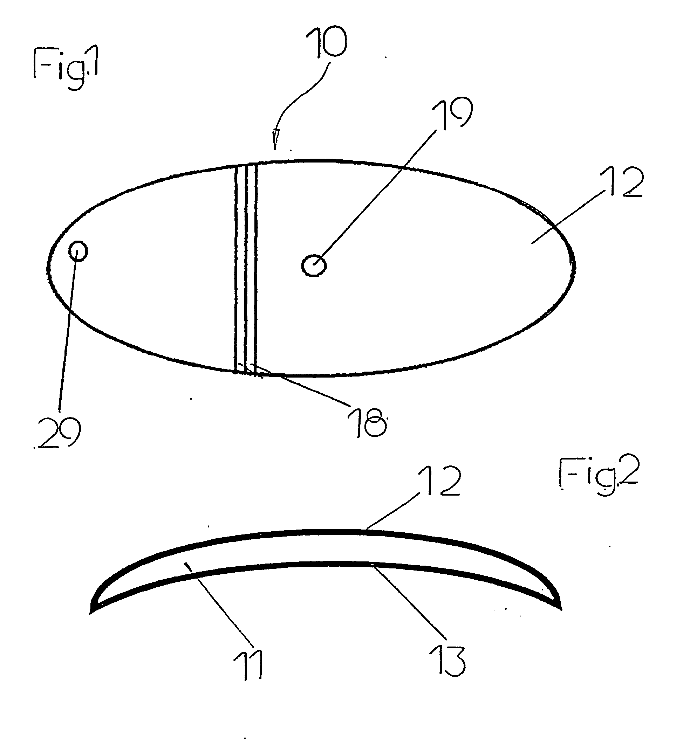

Gemäss Fig. 1 umfasst die erfindungsgemässe Pumpe 10 ein Gehäuse 11, in dem die Bestandteile der Pumpe 10 aufgenommen sind. Das Gehäuse 11 ist aus zwei gleichen Halbschalen, einer oberen Schale 12 und einer unteren Schale 13 gebildet, die über ihre freien Umfangsränder (Rand der oberen Schale 14, Rand der unteren Schale 15) miteinander in Eingriff stehen, d.h. verbunden sind. Die Verbindung ist flüssigkeitsdicht, so dass die Bestandteile der Pumpe 10 gegen Flüssigkeitszutritt abgedichtet geschützt sind. Die Ränder 14, 15 der oberen Schale 12 und unteren Schale 13 umschreiben eine Elypse, während die äusseren Flächen 16, 17 der Schalen 12, 13 von ihren Mittelpunkten aus in Längs- und Querrichtung der Schalen 12, 13 radiusförmig zu den Rändern 14, 15 verlaufen. Dadurch entsteht ein vergleichsweise dünnes Gehäuse 11 mit von seinem Mittelpunkt allseits nach aussen abnehmender Bemessung, d.h. mit fliessenden Konturen, die einen wesentlichen Beitrag zur Beseitigung der aus dem Stand der Technik bekannten, durch die Zylinderform der bekannten Pumpen verursachten, kosmetischen Probleme leistet. Zur vollständigen Beseitigung der kosmetischen Probleme ist das Gehäuse 11 der Pumpe 10 bogenförmig verformt, wobei die Verformung während der Implantation entsprechend der Form oder Kontur der Implantationsstelle vorgenommen wird. Implantiert werden Pumpen bei Menschen im Bauchbereich, zwischen Muskel und Haut, d.h. im subkutanen Fettgewebe, das das erfindungsgemässe Gehäuse ohne Ausbuchtungen nach aussen, d.h. vom Implantationsort weg, aufnimmt. Die obere Schale 12 weist Dehnungsfugen 18 auf, die die bogenförmige Verformung, d.h. die Anpassung des Gehäuses 11 an die Form eines Implantationsortes begünstigen. Dabei erfährt die obere Schale 12 eine Dehnung, während die untere Schale 13 gestaucht wird. Normalerweise können die Stauchungen ohne Kompensationsmittel vollzogen werden, sollte es jedoch notwendig oder zweckmässig sein, Stauchungen zu kompensieren oder zu erleichtern, so können auch an der unteren Schale Kompensationsfugen vorgesehen sein. Fugen 18 verlaufen vorzugsweise quer zur Längsachse, von Rand zu Rand der Schalen und sind so bemessen, dass sie sich dehnen oder zusammenschieben lassen. Gehäuse 11 werden bevorzugt aus Kunststoffen, vorzugsweise UV (ultra-violettes Licht) härtenden Kunststoffen gefertigt. UV härtende Kunststoffe für Gehäuse 11 sind vorteilhaft, weil die Gehäuse 11 vor oder während des Implantationsvorganges leicht plastifiziert, der Form der Implantationsstelle angepasst und anschliessend wieder UV gehärtet werden können.

Die im Gehäuse 11 der Pumpe 10 aufgenommenen Bestandteile umfassen einen Tank 20 mit Einstechdorn 19 zur Tankbefüllung, eine Batterie 21, eine elektronische Steuerung 22, eine nach dem piezzoelektrischen Prinzip arbeitende Fördereinrichtung 23 (folgend Piezzopumpe 23 genannt), Fördereinrichtungen, z.B. Schläuche, zur Förderung des flüssigen Tankinhaltes vom Tank 20 zur Piezzopumpe 23 und von dort zu einem Sideport 29 und elektrische Leitungen, die die Batterie 21 mit der Steuerung 22 und letztere mit der Piezzopumpe 23 verbinden.

Der Tank 20 entspricht in seiner körperlichen Ausgestaltung dem inneren Hohlraum des Gehäuses 11, ist jedoch entlang seiner Längsachse durch eine zur Längsachse rechtwinklig verlaufende Umfangskante 26 verkürzt, damit auch die übrigen Pumpenbestandteile im Innenraum des Gehäuses 11 Platz finden. Der Tank 20, d.h. seine Wandungen sind aus einer biegsamen, d.h. flexiblen, säuren- und laugenbeständigen Kunststoffolie gebildet, die einseits Noppen (nicht gezeigt) trägt, folgenden Noppenfolie genannt. Nach Fertigung des Tankes 20 bildet die mit Noppen versehene Oberfläche der Folie die innere Oberfläche der Tankwandungen. Die Noppen verhindern, dass bei leerem Tank 20 die inneren Oberflächen der Wandungen zusammenkleben. Umgeben ist der Tank 20 von einem Druckkörper 27. Der Druckkörper 27 ist aus einem komprimier- und expandierenden Kunststoffschaum gebildet. Seine äussere Form entspricht der Form des Hohlraumes des Gehäuses 11, während seine innere Form so bemessen ist, dass der Tank 20 mit aneinander anliegenden oberer und unterer Tankwandung Aufnahme findet. Wird der Tank befüllt, dehnt er sich aus und die obere und untere Tankwandung komprimiert den Schaumstoff gegen die innere Oberfläche des Gehäuses 11. Durch diese Kompression übt der Schaumstoff des Druckkörpers 27 auf den Tank 20 einen Gegendruck aus, der sich mit Entleerung des Tankes 20 abbaut. Mit der allseitigen Druckbelastung des Tanks 20 wird erreicht, dass er sich vollständig entleert und sichergestellt ist, dass die Pumpe 10 in jeder Lage fluidfördernd arbeitet.

Befüllt wird der Tank 20 über ein Ventil 19 (auch Einstechdorn 19 genannt), das ausgehend von der oberen Schale 12 die Schale 12, den Druckkörper 27 und eine Tankwandung durchgreifend in dem Tank 20 ausmündet. Bei dem Einstechdorn 19 handelt es sich grundsätzlich um ein Ventil, das zur Befüllung in einer Richtung geöffnet werden kann, nach Befüllung geschlossen gehalten wird. Der Tank 20 weist einen Auslass 30 auf, der beispielsweise über eine Förderleitung, z.B. einen Schlauch, mit der Eingangsseite 31 der Piezzopumpe 23 verbunden ist. Damit wird der Eingangsseite 31 der Piezzopumpe 23 ständig Fluid angeboten.

Die Piezzopumpe 23 ist ein aus piezzoelektrischen Kristallen aus mindestens zwei Rohrabschnitten 23a und 23b gefertigtes Rohr, wobei an jedem Rohrabschnitt 23a, 23b über nicht näher dargestellte Leitungen individuell ein Strom oder Spannung anlegbar ist. Bekannt ist, dass piezzoelektrische Kristalle auf eine angelegte elektrische Spannung oder einen angelegten Strom durch Veränderung ihrer Gitter reagieren. Wird beispielsweise eine Spannung an den Kristall angelegt, dehnt sich das Gitter, wird der Kristall spannungslos gestellt, zieht sich das Gitter zusammen. Dieses Effektes bedient sich die Piezzopumpe 23, indem an jeden der Rohrabschnitte 23a, 23b ein Strom oder eine Spannung anlegbar und abstellbar ist. Wird ein Strom oder eine Spannung an die Rohrabschnitte 23a, 23b angelegt, so erweitern sie sich einschliesslich ihrer Bohrungen (Bohrung geht auf), wird der Strom oder die Spannung abgestellt, ziehen sie sich einschliesslich Bohrung zusammen (Bohrung geht zu). Zur Erzeugung eines Pumpvorganges sind die Rohrabschnitte 23a, 23b elektrisch gegenläufig geschaltet. Wird an den Rohrabschnitt 23a ein Strom angelegt, erfolgt die beschriebene Erweiterung, d.h. die Bohrung des Rohrabschnittes 23a ist offen und kann ein Fluid aufnehmen. Während der Aufnahme liegt am Rohrabschnitt 23b kein Strom an, der Rohrabschnitt 23b ist geschlossen. Ist die Fluidaufnahme (Befüllung)in Rohrabschnitt 23a abgeschlossen, wird der an Rohrabschnitt 23a angelegte Strom abgestellt und ein Strom an Rohrabschnitt 23b angelegt, dabei schliesst sich Rohrabschnitt 23a und Rohrabschnitt 23b öffnet sich, das Fluid wird aus der Bohrung des Rohrabschnittes 23a abgefördert und durchströmt die Bohrung des Rohrabschnittes 23b (Abförderung). Ist die Abförderung abgeschlossen, wird der Rohrabschnitt 23b stromlos gestellt und Rohrabschnitt 23a mit Strom beaufschlagt, woraufhin sich der Pumpvorgang wiederholt, dabei wirkt Rohrabschnitt 23b als in Abströmrichtung letzter von nacheinander angeordneten Abschnitten in geschlossenem Zustand als Rückschlagventil. Die Abschaltung des Stromes des Rohrabschnittes 23a läuft der Aufschaltung des Stromes auf Rohrabschnitt 23b kurzzeitlich voraus, so dass durch die Kontraktion der Bohrung des Rohrabschnittes 23a gegen den noch geschlossenen Rohrabschnitt 23b Druck auf das Fluid ausgeübt wird, so dass das Fluid unter höherem Druck aus dem Rohrabschnitt 23b abströmt, als es in den Rohrabschnitt 23a eingeströmt ist. Die Erfindung ist nicht auf eine Piezzopumpe 23 mit zwei Rohrabschnitten 23a, 23b beschränkt. Piezzopumpen nach der Erfindung können aus mehr als zwei Rohrabschnitten gebildet sein. Dies ist dann zweckmässig, wenn höhere Drücke des Fluids - der Druck steigt in Förderrichtung mit jedem Rohrabschnitt - gefordert sind. Hinzutritt, dass mit steigender Anzahl Rohrabschnitten grössere und genauere Fluidmengen abgegeben werden können.

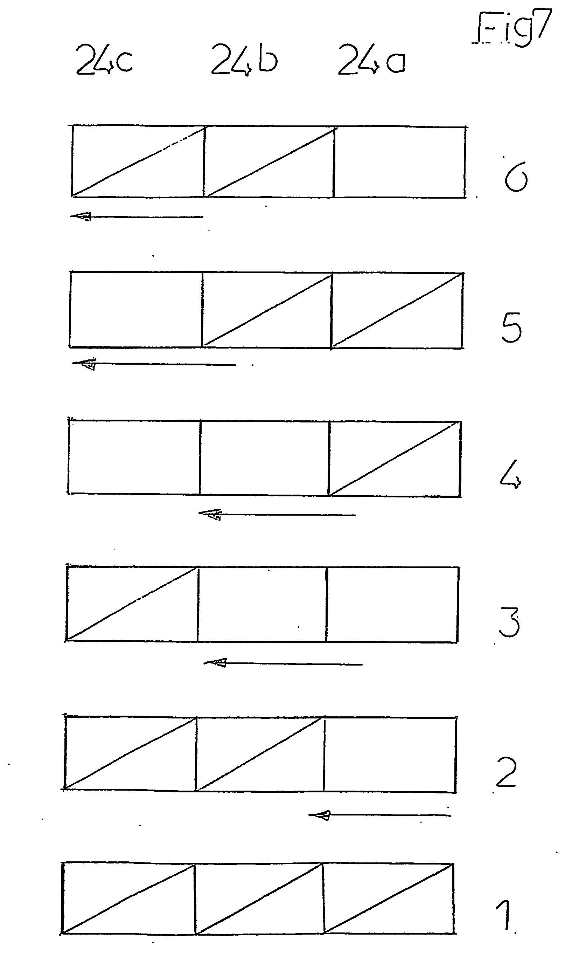

Folgend wird in Zusammenhang mit Fig. 7 der Funktionsablauf einer mehrstufigen, im dargestellten Fall dreistufigen Piezzopumpe 23 beschrieben. Die Piezzopumpe 23 gemäss Fig. 7 umfasst drei Rohrabschnitte 24a, 24b, 24c (folgend Abschnitte 24a, 24b, 24c genannt), die jeder für sich piezzoelektrisch, beispielsweise durch Anlegen eines Stromes aktivierbar und deaktivierbar sind. Dargestellt sind 6 Schaltungen (Schaltungen 1 bis 6). Jeder Abschnitt 24a, 24b, 24c, ist als Rechteck dargestellt, wobei ein Rechteck ohne eingezeichnete Diagonale bedeutet, dass der Abschnitt offen, ein Rechteck mit eingezeichneter Diagonale besagt, dass der Abschnitt geschlossen ist. Die gezeigten Pfeile deuten die Fliessrichtung an.

In Schaltung 1 liegt an der Pumpe 23 und auch an keinem ihrer Abschnitte 24a, 24b oder 24c ein Strom oder eine Spannung an, die Pumpe ist ohne Strom folglich geschlossen, indem alle Abschnitte geschlossen sind. Dies ist ein nach der Erfindung ausgebildetes Sicherheitsmerkmal, indem bei Ausfall der Batterie 21 die geschlossenen Abschnitte, ein Eindringen von Körperflüssigkeit in den Tank 20 verhindern.

Bei Schaltung 2 wird an Abschnitt 24a ein Strom angelegt, der Abschnitt 24a öffnet und wird vom Tank 20 her befüllt. Die Abschnitte 24b und 24c sind geschlossen.

In Schaltung 3 wird der Abschnitt 24a und 24b mit Strom beaufschlagt, der Abschnitt 24b öffnet und bildet mit dem offenen Abschnitt 24a einen vergrösserten Füllraum, der ergänzend zur Füllmenge in Abschnitt 24a mit einer dem Abschnitt 24b entsprechenden Menge vom Tank 20 befüllt wird. Die Befüllungen in Schaltungen 2 und 3 erfolgen überwiegend durch Ansaugen, wobei der Ansaugvorgang durch die Öffnung der Abschnitte 24a, 24b ausgelöst wird. Während des zweistufigen Ansaugevorganges sind die Piezzopumpe 23 und Tank 20 durch den in Strömungsrichtung letzten geschlossenen Abschnitt (24c) gegen unter Druck stehende Körperflüssigkeit, gesichert. Ein Druckaufbau entsteht, indem während des Schliessvorganges von 24a (Schaltung 4) die Befüllmenge von 24a und 24b gegen den geschlossenen Abschnitt 24c gedrückt wird, indem der Abschnitt 24c kurzzeitig verspätet zum Abschnitt 24a öffnet.

In Schaltung 4 ist der Abschnitt 24a geschlossen, die Abschnitte 24b und 24c sind offen. Die ursprünglich in Abschnitt 24a und Abschnitt 24b aufgenommene Füllmenge wechselt unter höherem Druck stehend in die Abschnitte 24b und 24c, wobei eine erste Teilmenge der Befüllung abgefördert wird.

In Schaltung 5 schliesst der Abschnitt 24b, der Schliessvorgang drückt die zweite Teilmenge aus der Piezzopumpe 23 aus.

Bei Schaltung 6 ist Abschnitt 24c geschlossen und Abschnitt 24a wieder geöffnet, die Pumpe ist in die Schaltung 2 zurückgekehrt, für einen nächsten Pumpvorgang, bestehend aus Befüllen (Schaltung 2 und 3), Druckaufbau (Schaltung 3, Schliessung 24a und Schaltung 4, kurzzeitig verspätete Öffnung), Abförderung (Schaltung 4 und 5), Befüllen (Schaltung 6, identisch Schaltung 2).

Abweichend vom Stand der Technik, der die Pumpfunktion mechanischen Einrichtungen (Rollenpumpen) überträgt, geht die Erfindung davon abweichend den Weg, die Pumpfunktion einem aus mindestens zwei Rohrabschnitten bestehenden piezzoelektrisch aktivierbaren und deaktivierbaren Rohr zu überbinden. Rohre dieser Betriebsart sind verschleissfrei, können mit niedrigerem Energieaufwand als Pumpen mit mechanischen Fördereinrichtungen betrieben werden und Pumpen nach der Erfindung zeichnen sich dadurch aus, dass sie deutlich leichter als bekannte Pumpen sind. Besonders vorteilhaft ist, dass die Genauigkeit einander folgender Fördermengen über 99% beträgt. Diese hohe Fördergenauigkeit zieht nach sich, dass im Falle der Verabreichung von Medikamenten je Fördervorgang kleinere Mengen höherer Konzentration, als mit Pumpen nach dem Stand der Technik, verabreicht werden können.

Ein Rohr verbindet das abstromseitige Ende der Pumpe 23 mit einem Anschluss 29, folgend Sideport 29 genannt. Der Sideport 29 durchgreift die untere Schale 13 und an ihn wird der ausserhalb des Gehäuses 11 vorgesehene Katheter 25 mit seinem aufstromseitigen Ende A angeschlossen, während das abstromseitige Ende B je nach Indikation, beispielsweise in den Spinalraum oder die Blutbahn eines Rezipienten fördert. Die untere Schale 13 weist auf ihrer äusseren Fläche 17 eine Ausnehmung wellenförmigen Verlaufes auf, in die der Katheter 25 verlegt ist. Damit kann der Katheter 25 knick- und spannungsfrei an die Pumpe 23 angeschlossen werden. Ferner steht damit ein Schlauchvorrat zur Verfügung, der bei Körperbewegungen zugbedingte Längenausdehnungen des Katheters 25 zur Meidung von Katheterabrissen ausgleicht. Das Sideport 29 ist einmal als Katheteranschluss an unterer Schale 13 und als in den Katheteranschluss förderndes Ventil ausgebildet, das die obere Schale 12 durchgreift, in das von aussen bei laufender Pumpe 23 zum Fluidstrom zusätzliche Flüssigkeitsmengen, z.B. medikamentöse Zusammensetzung per Injektion (Spritze mit Nadel) zugebbar sind. Bei abgeschalteter Pumpe 23 kann das Sideport 29 zur Spülung des Katheters und für Dichtigkeitsprüfungen verwendet werden.

Für den Einstichdorn 19 und das gleichwirkende Sideport 29 sind Ventile bevorzugt, die aus medizinisch verwendbarem Titan mit einer Ventilfunktion vollziehenden Silikonfüllung bestehen. Zur Befüllung wird die Silikonfüllung beispielsweise mit einer Hohlnadel durchstossen, wobei nach Befüllung die Hohlnadel zurückgezogen wird und sich die Durchstossöffnung durch die Silikonfüllung schliesst. Ventile dieser Art sind einfachen Aufbaues ohne bewegliche Teile und zeichnen sich durch eine hohe Funktionssicherheit aus.

Die implantattaugliche Batterie 21 ist über eine elektronische Steuerung 22 (auch Steuereinheit, oder Steuerung 22 genannt) mit der Piezzopumpe 23 elektrisch verbunden. Die Aufgabe der Steuerung ist, Strom aus der Batterie 21 der Piezzopumpe 23 impulsförmig zuzuleiten. Die zeitliche Abfolge zwischen den Impulsen, die das Auf- und Abschalten des Stromes (folgend gesamthaft Pumpimpuls genannt) und einem folgenden Pumpimpuls wird durch einen Uhrenquarz bestimmt, der nach Massgabe bestimmter, jeweils aufsummierter Schwingungen über das Steuergerät die Impulse auslöst. Diese Schwingungsanzahlen sind abrufbar und veränderbar auf einer Datenspeichereinrichtung (Microchip) gespeichert. Die Häufigkeit pro Zeiteinheit, mit der die Pumpimpulse ausgelöst werden, wird folgend Traktfrequenz genannt. Einbezogen in die Programmierung des Steuergerätes 22, d.h. des Chips, wird die maximale Menge, die eine Piezzopumpe 23 während eines Pumpimpulses fördern kann, diese wird als feste, unveränderbare Grösse auf den Chip eingegeben. Dies bedeutet, dass jede Piezzopumpe 23 während ihrer Herstellung einzeln kalibriert, d.h. einzeln eingemessen wird, wodurch jede Piezzopumpe 23 zu einem identifizierbaren, patientenspezifischen Unikat mit eigenen technischen Daten wird. Die maximale Fördermenge als konstante Grösse in Beziehung gesetzt zur Taktfrequenz ermöglicht dem Chip, Angaben zur Fördermenge, Restmenge etc. zu errechnen. Mit 32 ist ein Magnetschalter bezeichnet, der nur mittels eines Codes zu aktivieren oder zu deaktivieren ist und in einem der beiden Zustände den Zugriff auf den Chip des Steuergerätes 22 zur Aenderung von Daten freigibt. Vom Chip der elektronischen Steuerung 22 sind auch nach Implantation der Pumpe 10 als Informationen abrufbar das Medikament, die ursprüngliche Füllmenge, die Menge einer Einzelabförderung (Dosis) pro Pumpimpuls, die Taktfrequenz, die Gesamtmenge des abgeförderten Medikamentes, die Restmenge im Tank 20, die Laufzeit der Pumpe 10, der Zustand der Batterie, eine Identifikation (Registrierung) der Pumpe 10 und sofern vom Arzt programmiert die Anzahl Bolus (Medikamentenportion), die sich ein Patient zusätzlich vermittels der Pumpe 10 zu den von der Pumpe 10 programmgemäss geförderten Portionen verabreicht hat, beispielsweise ergänzt um Datum und Zeitpunkt der zusätzlichen Verabreichungen. Die vorstehenden Informationen werden seitens des Chips teils errechnet oder dokumentiert. Errechnet wird beispielsweise die Restmenge im Tank 10, deren Stand für Notsignale zur Tankneufüllung und Warnung vor vermehrtem Fluidverbrauch, gesamte abgegebene Menge etc.

Die elektronische Steuerung 22 der Pumpe 10 wirkt mit einer Datenverarbeitungseinrichtung 33 (folgend Datenverarbeiter 33 genannt) zusammen, der über einen freien Lesekopf 34 mit der elektronischen Steuerung 22, d.h. ihrem datentragenden Speicher oder Chip kommuniziert. Der Datenverarbeiter 33, beispielsweise ein herkömmlicher Personalcomputer, steht dem Arzt zur Verfügung, während der Patient den Lesekopf 34, der nicht implantiert ist, verfügbar hat. Auf dem Datenverarbeiter 33 werden vermittels eines Programmes alle therapie- und pumpenbetriebsspezifischen Werte festgelegt, gespeichert und über den Lesekopf 34 auf den Chip der Steuereinrichtung übertragen. Die Übertragung erfolgt, indem der Patient den Lesekopf 34 auf die implantierte Pumpe 10 hält, d.h. er bringt Chip und Lesekopf 34 in kommunikativen Eingriff miteinander. In umgekehrter Richtung kann der Lesekopf Daten vom Chip aufnehmen und an den Datenverarbeiter 33, zwecks Überarbeitung, zurückreichen. Nach erster Programmierung des Chips und Implantation der Pumpe 10 bleiben Steuereinrichtung 22 und Datenverarbeiter 33 über den Lesekopf 34 auf Abruf in Verbindung, dabei kann z.B. der Arzt Daten vom Chip über den Lesekopf 34 abrufen, vergleichen, für therapeutische Zwecke verändern und dem Chip wieder zuleiten. Bevorzugt nach der Erfindung wird die Datenübertragung von Datenverarbeiter 33 zum Lesekopf 34 über Telefonleitungen oder ähnliche Fernsprechübertragungseinrichtungen in Anwendung des sogenannten Tonwahlverfahrens vorgenommen, das Daten und Töne in verschiedenen Tonlagen zuordnet und überträgt. Diese Art der Datenübertragung ist über grosse Distanzen möglich, so dass der Arzt therapeutische Massnahmen auch bei grosser distanzmässiger Trennung zum Patienten vornehmen kann.

Ist die Bolusabgabe nach Massgabe der ärztliche Vorschrift programmiert, dann kann der Lesekopf 34 mit einem Signalgeber 35, der nur die Bolusabgabe auslöst, kombiniert sein.

Die Pumpe 10 nach der Erfindung kennzeichnet sich wie beschrieben durch zwei Betriebsarten. Die erste Betriebsart (Mode 1) ist die Abgabe bestimmter Mengen im Rahmen einer vorgegebenen Taktfrequenz, die zweite Betriebsart (Mode 2) umschliesst die erste, bedarfsweise ergänzt um die Abgabe von Zusatzmengen (Bolus). Bei medizinischer Anwendung der Pumpe 10 nach der Erfindung stützt sich Mode 1 bezüglich Mengenabgaben, Häufigkeit der Abgaben, Zusammensetzung der Medikamente etc. auf die Erfahrungen des Arztes ab, der danach strebt, seiner therapeutischen Zielsetzung möglichst nahe zu kommen. Sind Insuffizienzen, z.B. der Blutzuckergehalt eines Diabeteskranken nicht schwankend, so reicht es nach Massgabe der ärztlichen Verordnung (programmierte Insulinabgabe), die Pumpe 10 ohne Bolus-Abgaben zu betreiben. Sind Insuffizienzen schwankend, und lösen Schwankungsausschläge seitens des Patienten, d.h. subjektiv empfundene Störungen aus, dann ist Mode 2 bevorzugt, da mit Mode 2 die Verordnung des Arztes nach Mode 1 zur Beseitigung der Störungen durch den Patienten ergänzt oder modifiziert werden kann. Geht man davon aus, dass Mode 1 ärztlichen Kenntnissen und allgemein anerkannten Erfahrungsregeln folgt, also eine objektivierte Therapievorschrift umfasst, so tritt bei Mode 2 ein subjektives Empfindungselement hinzu, dass keine Rückschlüsse auf die Höhe der Schwankungsausschläge zulässt. Um dem entgegenzuwirken, kann für die Bolusabgabe ein Schwankungsausschläge messender Sensor (nicht gezeigt) vorgesehen sein, der Ausschläge von Einstellwerten nach Mode 1 misst und bei dern Überschreiten oder Unterschreiten der Pumpe 10, d.h. dem Chip des Steuergerätes 22, den Befehl erteilt, Zusatzmengen/Mindermengen zur Kompensation der Schwankungsausschläge zu fördern. Sensoren können z.B. in der Blutbahn bei Diabetes implantiert sein oder, wo angezeigt, auf dem Körper getragen werden.

## Patentansprüche

1. Infusionspumpe umfassend ein implantierbares Gehäuse, einen im Gehäuse aufgenommenen, mit einem Fluid befüllbaren Tank mit Einstechdorn und Auslass, einem Katheter, einer zwischen Tank und Katheter angeordneten, über eine Batterie getriebene Fördereinrichtung, deren Fördermenge über eine elektronische Steuerung einstellbar ist, wobei die Fördereinrichtung (23) nach piezzoelektrischem Prinzip wirkend ausgebildet ist, **dadurch gekennzeichnet, dass** die äussere Fläche 16 der oberen Schale (12) des Gehäuses (11) Dehnungsfugen (18) aufweist.

2. Infusionspumpe nach Anspruch 1, **dadurch gekennzeichnet, dass** die Fördereinrichtung (23) ein aus piezzoelektrischen Kristallen bestehendes, mindestens aus zwei Rohrabschnitten (23a) und (23b) gebildetes Rohr (Piezzopumpe 23) ist, an dessen Rohrabschnitte (23a) und (23b) ein elektrischer Strom oder eine Spannung anlegbar ist.

3. Infusionspumpe nach Anspruch 1 oder 2, **dadurch gekennzeichnet, dass** die Piezzopumpe (23) einends in einen Sideport (29) unter Verschluss eines Rohrabschnittes (23a) oder (23b) fördert und anderenends an den Sideport (29) ein Katheter (25) anschliessbar ist.

4. Infusionspumpe nach einem der Ansprüche 1 bis 3, **dadurch gekennzeichnet, dass** die äussere Fläche (17) der unteren Schale (13), ausgehend vom Sideport (29), eine wellenförmige Ausdehnung aufweist, in die der an das Sideport (29) angeschlossene Katheter (25) aufgenommen ist.

5. Infusionspumpe nach einem der Ansprüche 1 bis 4, **dadurch gekennzeichnet, dass** der Tank (20) aus einer biegsamen Folie gebildet ist und auf seiner inneren Oberfläche Noppen aufweist.

6. Infusionspumpe nach einem der Ansprüche 1 bis 5, **dadurch gekennzeichnet, dass** der Tank 20 in einem aus einem komprimierbaren Kunststoffschaum gebildeten Druckkörper (27) aufgenommen ist.

7. Infusionspumpe nach einem der Ansprüche 1 bis 6, **dadurch gekennzeichnet, dass** zwischen Batterie (21) und Piezzopumpe (23) eine Steuerung (22) zur Aktivierung und Deaktivierung der Rohrabschnitte (23a) und (23b) der Piezzopumpe (23) vorgesehen ist.

8. Infusionspumpe nach einem der Ansprüche 1 bis 7, **dadurch gekennzeichnet, dass** Aktivierung oder Deaktivierung durch Anlage eines Stromes oder einer Spannung an die Rohrabschnitte (23a), (23b) erfolgt.

9. Infusionspumpe nach einem der Ansprüche 1 bis 8, **dadurch gekennzeichnet, dass** die Steuerung (22) vermittels eines Datenverarbeiters (33) und einem mit dem Datenverarbeiter (33) kommunizierenden Lesekopf (34) programmierbar ist.

10. Infusionspumpe nach einem der Ansprüche 1 bis 9, **dadurch gekennzeichnet, dass** die Kommunikation über Telefonleitungen oder ähnliche Fernsprechübertraguhgseinrichtugen erfolgt.

11. Infusionspumpe nach einem der Ansprüche 1 bis 10, **dadurch gekennzeichnet, dass** Daten nach dem Tonwahlverfahren aufgeschlüsselt und übertragen sind.

12. Infusionspumpe nach einem der Ansprüche 1 bis 11, **dadurch gekennzeichnet, dass** der Lesekopf (34) einen Signalgeber (35) zur Abgabe eines Bolus aufweist.

## Claims

1. Infusion pump comprising an implantable housing, a reservoir which is received in the housing, can be filled with a fluid and has a piercing mandrel and an outlet, a catheter, a battery-powered delivery device which is arranged between the reservoir and the catheter and of which the delivery rate can be adjusted via an electronic control unit, the delivery device (23) being constructed so as to operate piezoelectrically, **characterised in that** the outer surface (16) of the upper shell (12) of the housing (11) comprises expansion joints (18).

2. Infusion pump according to claim 1, **characterised in that** the delivery device (23) is a tube (piezo pump 23) consisting of piezoelectric crystals and made up of at least two tube portions (23a) and (23b), and an electric current or a voltage can be applied to the tube portions (23a) and (23b) of said tube.

3. Infusion pump according to either claim 1 or claim 2, **characterised in that** the piezo pump (23) feeds into a side port (29) at one end, sealing a tube portion (23a) or (23b), and at the other end a catheter (25) can be attached to the side port (29).

4. Infusion pump according to any one of claims 1 to 3, **characterised in that** the outer surface (17) of the lower shell (13), starting from the side port (29), comprises a wave-shaped extension in which the catheter (25) attached to the side port (29) is received.

5. Infusion pump according to any one of claims 1 to 4, **characterised in that** the reservoir (20) is formed from a flexible film and comprises naps on the inner surface thereof.

6. Infusion pump according to any one of claims 1 to 5, **characterised in that** the reservoir (20) is received in a pressure member (27) formed from a compressible synthetic foam.

7. Infusion pump according to any one of claims 1 to 6, **characterised in that** a control unit (22) for activating and deactivating the tube portions (23a) and (23b) of the piezo pump (23) is provided between the battery (21) and the piezo pump (23).

8. Infusion pump according to any one of claims 1 to 7, **characterised in that** activation or deactivation is carried out by applying a current or a voltage to the tube portions (23a), (23b).

9. Infusion pump according to any one of claims 1 to 8, **characterised in that** the control unit (22) is programmable via a data processor (33) and a read head (34) which communicates with the data processor (33).

10. Infusion pump according to any one of claims 1 to 9, **characterised in that** the communication takes place via telephone lines or similar telephone transmission means.

11. Infusion pump according to any one of claims 1 to 10, **characterised in that** data are coded and transmitted using tone dialling.

12. Infusion pump according to any one of claims 1 to 11, **characterised in that** the read head (34) has a signal emitter (35) for administering a bolus.

## Revendications

1. Pompe à perfusion comprenant un boîtier implantable, un réservoir contenu dans le boîtier et pouvant être rempli avec un fluide muni d'une pointe d'injection et d'une sortie, d'un cathéter, d'un dispositif de refoulement disposé entre entre le réservoir et le cathéter et alimenté par une batterie, dont le débit est réglable via un contrôleur électronique, le dispositif de refoulement (23) étant configuré pour fonctionner par un principe piézoélectrique, **caractérisé en ce que** la surface extérieure 16 de la coque supérieure (12) du boîtier (11) comporte des joints de dilatation (18).

2. Pompe à perfusion selon la revendication 1, **caractérisé en ce que** le dispositif de refoulement (23) est un tube (pompe piézo 23) réalisé de cristaux piézoélectriques, comprenant au moins deux portions de tube (23a) et (23b), aux portions de tube (23a) et (23b) duquel peut être appliqué un courant ou une tension électrique.

3. Pompe à perfusion selon la revendication 1 ou 2, **caractérisé en ce que** la pompe piézo (23) refoule d'un côté d'une ouverture latérale (29) lors de la fermeture d'une des portions de tube (23a) et (23b), et qu'un cathéter (25) peut être connecté de l'autre côté de l'ouverture latérale (29).

4. Pompe à perfusion selon l'une des revendications 1 à 3, **caractérisée en ce que** la surface extérieure (17) de la coque inférieure (13) comporte à partir de l'ouverture latérale (29), une extension ondulée dans laquelle se loge le cathéter (25) connecté à l'ouverture latérale (29).

5. Pompe à perfusion selon l'une des revendications 1 à 4, **caractérisée en ce que** le réservoir (20) est réalisé à partir d'un film souple et comporte des bossages sur sa surface interne.

6. Pompe à perfusion selon l'une des revendications 1 à 5, **caractérisée en ce que** le réservoir (20) est logé dans un corps de pression (27) formé de mousse en matière plastique compressible.

7. Pompe à perfusion selon l'une des revendications 1 à 6, **caractérisée en ce qu'**un contrôleur (22) pour l'activation et la désactivation des portions de tube (23a) et (23b) est disposé entre la batterie (21) et la pompe piézo (23).

8. Pompe à perfusion selon l'une des revendications 1 à 7, **caractérisée en ce que** l'activation ou la désactivation à lieu par application d'un courant ou d'une tension aux portions de tube (23a) et (23b).

9. Pompe à perfusion selon l'une des revendications 1 à 8, **caractérisée en ce que** le contrôleur (22) est programmable au moyen d'un dispositif de traitement de données (33) et d'une tête de lecture (34) communiquant avec le dispositif de traitement de données (33).

10. Pompe à perfusion selon l'une des revendications 1 à 9, **caractérisée en ce que** la communication est réalisée par une liaison téléphonique ou un dispositif de transmission téléphonique semblable.

11. Pompe à perfusion selon l'une des revendications 1 à 10, **caractérisée en ce que** des données sont codées et transmises selon le mode en fréquence vocale.

12. Pompe à perfusion selon l'une des revendications 1 à 11, **caractérisée en ce que** la tête de lecture (34) comporte un émetteur de signal (35) pour la délivrance d'un bolus.
